# EUROPEAN PATENT SPECIFICATION

(11) **EP 2 493 597 B1**
(45) Date of publication and mention of the grant of the patent: **29.03.2017**
(21) Application number: 10830479.1
(22) Date of filing: 29.10.2010
(51) Int. Cl.: B01D 61/28

(54) **MICROFLUIDIC DEVICE FOR BLOOD DIALYSIS**
MIKROFLUIDISCHE VORRICHTUNG FÜR HÄMODIALYSE
DISPOSITIF MICROFLUIDIQUE POUR LA DIALYSE DU SANG

(30) Priority: 29.10.2009 US 256093 P
(43) Date of publication of application: 05.09.2012
(73) Proprietor: The Charles Stark Draper Laboratory, Inc., Cambridge, MA 02139-3563 (US); The General Hospital Corporation, Boston, MA 02114 (US)
(72) Inventor: CHAREST, Joseph, L., Cambridge MA 02138 (US); BORENSTEIN, Jeffrey, T., Newton MA 02464 (US); ARNAOUT, M., Amin, Chestnut Hill MA 02467 (US)
(74) Representative: Somervell, Thomas Richard
(86) International application number: PCT/US2010/054602
(87) International publication number: WO 2011/059786

(56) References cited:
- EP-A1- 1 547 676
- WO-A1-02/076529
- US-A1- 2002 052 571
- US-A1- 2004 147 032
- US-A1- 2004 147 032
- US-A1- 2004 265 183
- US-A1- 2005 048 519
- US-A1- 2005 202 557
- US-A1- 2005 202 557
- US-A1- 2007 183 935
- US-A1- 2009 211 977

## Description

### RELATED APPLICATIONS

This application claims the benefit of and priority to United States Provisional Patent Application serial number 61/256,093, filed October 29, 2009.

### FIELD OF THE INVENTION

The present invention relates to microfluidic devices and methods of using such devices for filtering solutions.

### BACKGROUND OF THE INVENTION

Kidney or renal system failure, due to injury, disease, or other health issues, can cause various physiological problems. Frequently encountered problems include abnormal fluid levels in the body, abnormal levels of calcium, phosphate, and/or potassium, deranged acid levels, and anemia. Toxic end products of nitrogen metabolism (e.g., urea, uric acid, and creatinine) can accumulate in blood and tissues. In some instances, proteinuria (protein loss in the urine) and/or hematuria (blood loss in the urine) may occur. Over the long-term, kidney problems may have significant repercussions on cardiovascular disease and other diseases.

Patients suffering from kidney failure or reduced kidney function often rely on dialysis procedures to supplement and/or replace their kidney function. Dialysis removes excess water, waste, and toxins from the body that healthy kidneys would ordinarily remove on their own. The frequency and extent of dialysis treatment can depend on, for example, the extent of kidney dysfunction.

There are two types of dialysis procedures used to treat loss of kidney function: (i) hemodialysis and (ii) peritoneal dialysis. With hemodialysis, a patient is connected to a hemodialysis machine using catheters that are inserted into the patient's veins and/or arteries. The patient's blood is passed through the machine, where toxins, waste, and excess water are removed, and the blood is then returned to the patient. Hemodialysis is usually performed in dialysis centers, where the treatment entails dialysis for four hours three times a week. This sharply interferes with the patient's quality of life and ability to contribute to the community at large.

In peritoneal dialysis, a patient's peritoneal membrane is used as a filter, and toxins, waste, and excess water are drained in and out of the abdomen. Dialysate (a dialysis solution) is introduced into the patient's peritoneal cavity where it contacts the patient's peritoneal membrane. The toxins, waste, and excess water pass through the peritoneal membrane and into the dialysate via diffusion and osmosis. The used dialysate, containing the toxins, waste, and water, is then drained from the patient. The above steps may need to be repeated several times.

Like hemodialysis, peritoneal dialysis is inconvenient and leaves ample room for therapy enhancements to improve the patient's quality of life. For example, the process often requires significant manual effort on the part of the patient, and the patient generally needs to undergo multiple treatment cycles, each lasting about an hour.

For the above reasons, there is a need for improved filtration technology, particularly blood filtration technology that is more convenient for the patient. The present invention addresses these needs and provides other related advantages.

US2004/0265183 discloses forming structures having a pair of microfluidic channels in which a second channel has a width that spans a single first channel.

US2005/0202557 discloses forming first and second channels in two mold layers, which are manufactured through the same process such that the first and second channels are of similar widths.

WO02/076529 discloses a blood processing device in which a first plate has a number of first channels in a first direction for transporting blood, and a second plate has a number of second channels for transporting a gas. A gas-permeable membrane is arranged between the channels in the first and second plates.

EP1,547,676 discloses porous membrane microstructure devices, in which a porous membrane is arranged between corresponding microchannels formed in respective first and second ceramic or glass-ceramic plates.

US2009/0211977 discloses microfluidic transfer devices having arrangements of first and second channels of corresponding or similar widths.

### SUMMARY

The claimed invention is defined by the features of independent claims 1 and 10.

The claimed invention provides microfluidic devices and methods of filtering a liquid solution. The liquid solution may be for industrial applications or medical applications. For example, the microfluidic devices and methods are contemplated to provide particular advantages in blood dialysis and be applicable for mobile kidney augmentation devices. Microfluidic devices described herein contain one or more First Channels having dimensions that are particularly well suited for blood dialysis. It has been discovered that the First Channels characterized by particular ranges of height, width, and length provide superior fluid flow properties for blood filtration applications. One benefit of the First Channel features described herein is that they provide fluid shear rates that are contemplated to be amenable to, for example, red blood cells contained in blood. The First Channel features are also understood to be important for optimizing the amount of filtrate that passes through a filtration membrane separating the First Channel(s) from one or more Second Channels used to direct filtrate away from the purified blood.

Accordingly, the claimed invention provides a microfluidic device as set out in claim 1.

Another aspect of the claimed invention provides a method of filtering a liquid solution containing an analyte to provide a purified solution containing less analyte than said liquid solution, as set out in claim 10.

A further aspect, which is not a claimed aspect of the claimed invention, provides a wearable kidney augmentation device, comprising: (i) a filtration component comprising: (a) at least one First Channel and at least one Second Channel complementary to the at least one First Channel; and (b) a filtration membrane separating the at least one First Channel from the at least one Second Channel; wherein the at least one First Channel is configured to provide a fluid shear rate in the range of about 100 s<"1> to about 3000 s<"1> for blood at 37.0 °C; (ii) a first access conduit affording fluid communication with an input end of the at least one First Channel; (iii) a first return conduit affording fluid communication with an output end of the at least one First Channel; and (iv) a second return conduit affording fluid communication with an output end of the at least one Second Channel.

### BRIEF DESCRIPTION OF FIGURES

Figure 1 depicts a microfluidic device.
Figure 2 depicts a microfluidic device where a Second Channel (101) is fluidly connected to two First Channels (100) via a filtration membrane (102).
Figure 3 depicts a cross- sectional view of a microfluidic device having branched channels.
**Figure 4** is a graph showing the results of a computational model evaluating how changes in the length, width, and/or height of channels in a microfluidic device alter the calculated shear rate for fluid flowing through such channels.
**Figure 5** is a graph showing the results of a computational model evaluating how changes in the width and height of channels in a microfluidic device alter the calculated percentage of fluid that passes through a filtration membrane attached to the channels.
**Figure 6** is a table showing the results of a computational model illustrating how changes in the length, width, and/or height of channels in a microfluidic device alter the calculated shear rate for fluid flowing through the channels, as well as the percentage of fluid (i.e., filtrate) that passes through a membrane attached to the channels.

### DETAILED DESCRIPTION OF THE INVENTION

The present invention provides microfluidic devices and methods of filtering a liquid solution. The microfluidic devices and methods are contemplated to provide particular advantages in blood dialysis. As noted above, microfluidic devices described herein contain one or more First Channels having dimensions that are particularly well suited for blood dialysis. The particular combination of height, width, and length of the First Channels provides superior fluid flow properties for blood filtration. One benefit of the First Channel features described herein is that they provide fluid shear rates that are contemplated to be amenable to, for example, red blood cells contained in blood. Another benefit of the First Channel features is that they permit an optimal amount of filtrate to pass through a filtration membrane separating the First Channel(s) from one or more Second Channels used to direct filtrate away from the purified blood.

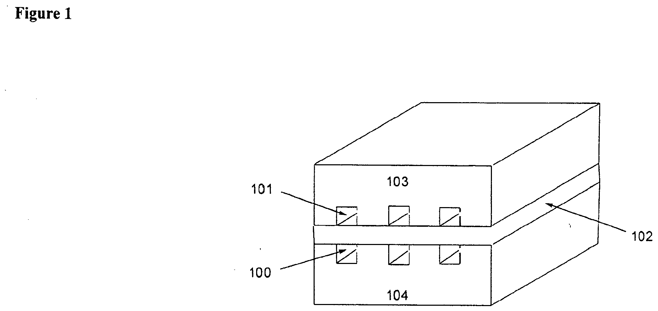

Certain aspects of the microfluidic devices are illustrated in Figure 1 showing an exemplary microfluidic device having a plurality of First Channels **100** separated from complementary Second Channels **101** by a filtration membrane **102.** The filtration membrane may be secured to support layers **103** and **104** through use of a chemical adhesive or by mechanical means. A liquid solution to be filtered is applied to an input end of the First Channels **100**. As the liquid solution passes through the First Channels **100,** analytes pass through the filtration membrane **102** into the Second Channel. The analytes that pass through the filtration membrane are collectively referred to as the filtrate, and are permitted to drain out of the Second Channels.

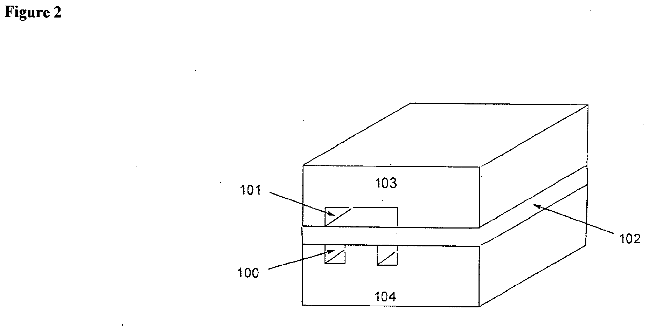

In certain embodiments, the microfluidic device may be configured according to the arrangement in Figure 2 where a plurality of First Channels **100** are separated from a complementary Second Channel **101** by a filtration membrane **102.** In this embodiment, a single Second Channel **101** is fluidly connected to two of the First Channels **100** via filtration membrane **102.** Further embodiments of microfluidic devices containing various arrangements for the First Channel(s) and Second Channel(s) are described in more detail below.

Microfluidic devices described herein are contemplated to be well suited for use in a kidney augmentation device (KAD). The KAD extracts filtrate from a patient's blood in order to augment the native kidney function and treat patients with kidney dysfunction. The device can be configured to augment native kidney function, including water balance (hyper/hypovolemia), ionic solute balance, small molecule excretion (blood urea nitrogen, creatinine, etc.), and middle molecule extraction (β₂-microglobulin, etc.). The KAD may be configured to provide a portable, wearable device that supplements or partially replaces conventional renal dialysis or filtration. Still further, the KAD may be configured to administer ions directly to the patient's bloodstream through a time-release mechanism built into the device.

Various aspects of the microfluidic device and KAD are described below in sections. Features described in one section are not meant to be limited to any particular section.

### I. Channel Features of the Microfluidic Device

Channels in the microfluidic device can characterized according to the their height, width, length, and channel geometry. It has been discovered that channels characterized by certain height, width, and lengths provide particular benefits for solution filtration applications, such as blood dialysis. The microfluidic devices described herein contain one or more First Channels and at least one Second Channel. Various features of the First Channel and Second Channel are described below.

### A. Features of the First Channel

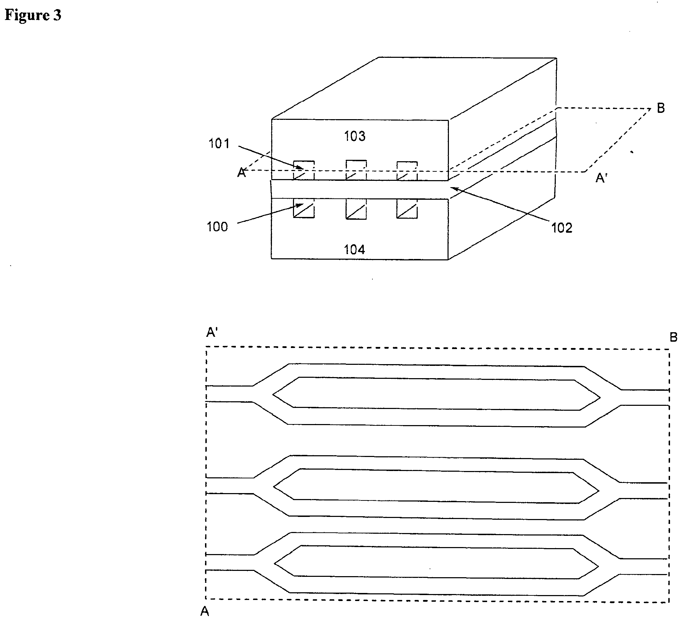

Microfluidic devices herein contain one or more First Channels, wherein each First Channel has a height in the range of about 50 µm to about 500 µm, a width in the range of about 50 µm to about 900 µm, and a length in the range of about 3 cm to about 20 cm. The First Channels in a microfluidic device may run approximately parallel to each other in the device, as depicted in Figure 1. Alternatively the one or more First Channels may be part of a network of interconnecting channels, as depicted in the cross-section view of a microfluidic device shown in Figure 3. The network of interconnecting channels may contain bifurcations or other geometries to direct fluid flow through the channels.

The First Channel(s) may have cross-sections that are round, rectangular, triangular, or other geometries. In certain embodiments, the First Channel(s) have cross-sections that are rectangular.

The channels can be molded in a polymeric material such as polystyrene, polycarbonate, polydimethylsiloxane, polymethylmethacrylate, cyclic olefin copolymer (e.g., ZEONOR), polysulfone, or polyurethane. For certain applications, the use of biodegradable or biocompatible materials, such as polyglycerol sebacate, polyoctanediol citrate, polydiol citrate, silk fibroin, polyesteramide, and/or polycaprolactone may be advantageous.

### Channel Dimensions

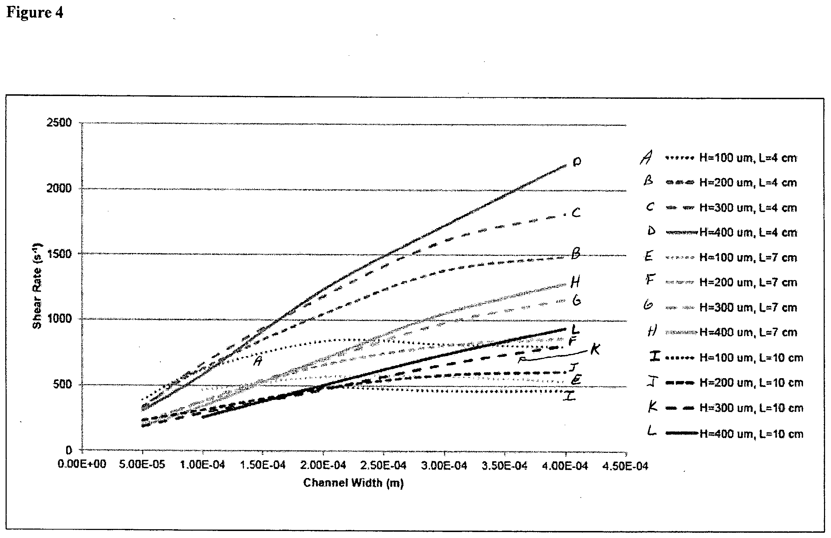

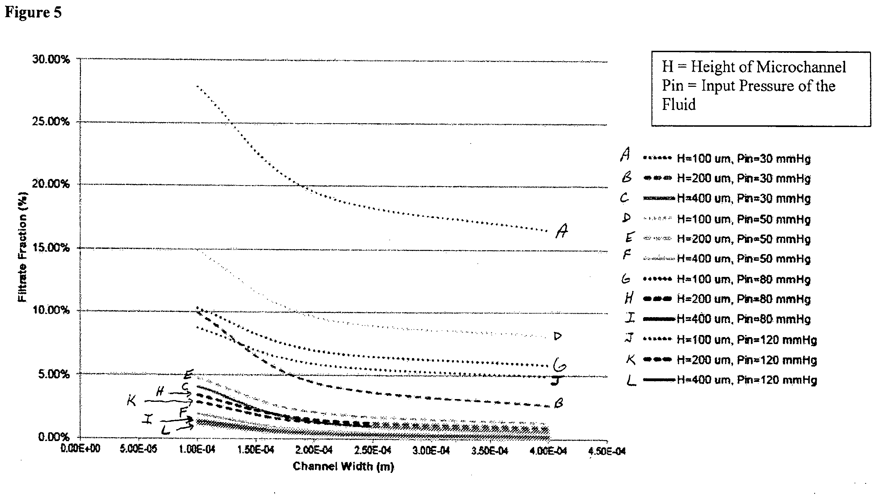

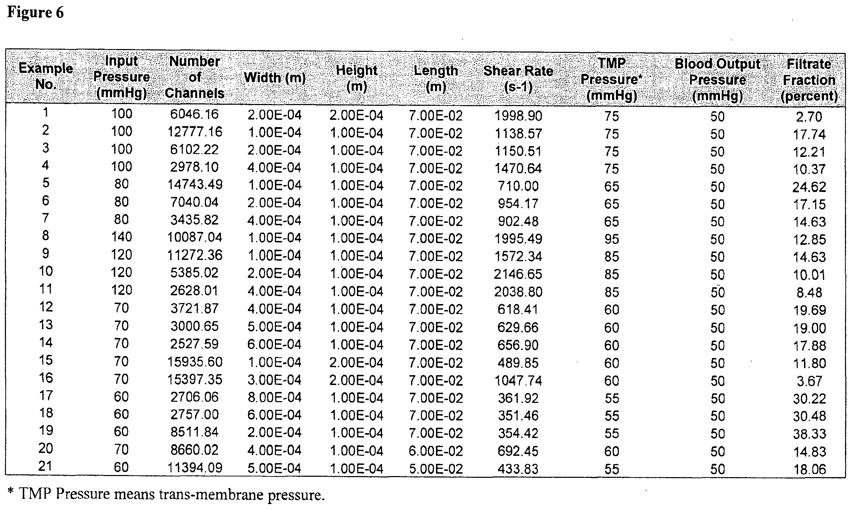

The dimensions of the First Channel(s) can be characterized according to the height, width, and length of the First Channel(s). It has been discovered that the certain channel dimensions characterized according to their height, width, and length provide superior performance for filtering solutions such as blood. Figure 4 shows the results of a computational model evaluating how changes to the length, width, and/or height of channels in a microfluidic device alter the calculated shear rate for fluid flowing through such channels. The calculations were performed to model conditions where the fluid pressure at the input end of the channels is 120 mmHg and the fluid pressure at the output end of the channels is 105 mmHg. Figure 5 shows the results of a computational model evaluating how changing the width and height of channels in a microfluidic device alters the calculated percentage of fluid that passes through a membrane attached to the channels. The calculations were performed to model conditions where the length of the channel is 7 cm and fluid pressure at the output end of the channels is 20 mmHg. Figure 6 provides further results of a computational model illustrating how changes in the length, width, and/or height of channels in a microfluidic device alter the calculated shear rate for fluid flowing through the channels, as well as the percentage of fluid (i.e., filtrate) that passes through a membrane attached to the channels.

Features of the First Channel(s) have been identified that are contemplated to provide superior conditions (e.g., acceptable shear rates for blood filtration, acceptable fluid pressure drop as fluid flows through the channels, and percentage of analyte that passes through a membrane attached to the channels) for blood filtration. For example, the First Channel(s) desirably have a height in the range of about 50 µm to about 500 µm. In certain other embodiments, the First Channel(s) have a height in the range of about 50 µm to about 400 µm, about 50 µm to about 300 µm, about 50 µm to about 150 µm, about 100 µm to about 200 µm, about 150 µm to about 250 µm, or about 80 µm to about 220 µm.

The First Channel(s) desirably have a width in the range of about 50 µm to about 900 µm. In certain other embodiments, the First Channel(s) have a width in the range of about 50 µm to about 150 µm, about 100 µm to about 200 µm, about 150 µm to about 250 µm, about 200 µm to about 300 µm, about 250 µm to about 350 µm, about 300 µm to about 400 µm, about 350 µm to about 400 µm, about 500 µm to about 600 µm, about 100 µm to about 500 µm, about 50 µm to about 2 mm, about 50 µm to about 1 mm, or about 0.5 mm to about 2 mm.

The First Channel(s) desirably have a length in the range of about 3 cm to about 20 cm. In certain other embodiments, the First Channel(s) have a length in the range of about 3 cm to about 10 cm, about 3 cm to about 5 cm, about 4 cm to about 6 cm, about 5 cm to about 7 cm, about 6 cm to about 8 cm, about 6.5 cm to about 7.5 cm, about 7 cm to about 9 cm, or about 7 cm.

The dimensions of the First Channel(s) can also be characterized according ratios of height versus width, and versus length. In certain embodiments, the First Channel(s) have a height to width ratio in the range of 1:1 to about 1:4, or about 1: to about 1:2. In certain embodiments, the First Channel(s) have height to length ratio in the range of 1:250 to about 1:800, or about 1:250 to about 1:400. In certain embodiments, the First Channel(s) have width to length ratio in the range of 1:250 to about 1:800, or about 1:250 to about 1:400.

The dimensions of the First Channel(s) can also be characterized by a combination of height, width, and length ranges described above, alone or in combination with the ratios of height versus width, and versus length described above. For example, in certain embodiments, each First Channel has a height in the range of about 50 µm to about 300 µm, a width in the range of about 50 µm to about 900 µm, and a length in the range of about 3 cm to about 10 cm. In certain embodiments, the First Channel(s) have one of the dimension set forth in Table 1 below.

**TABLE 1.**

| Example No. | Height (µm) | Width (µm) | Length (cm) |
|---|---|---|---|
| 1 | 80-120 | 380-420 | 6.5-7.5 |
| 2 | 80-120 | 380-420 | 6.9-7.1 |
| 3 | 80-120 | 380-420 | 7.0 |
| 4 | 80-120 | 300-400 | 6.5-7.5 |
| 5 | 80-120 | 200-300 | 6.5-7.5 |
| 6 | 80-120 | 100-200 | 6.5-7.5 |
| 7 | 80-120 | 90-120 | 6.5-7.5 |
| 8 | 100-200 | 300-400 | 6.5-7.5 |
| 9 | 100-200 | 200-300 | 6.5-7.5 |
| 10 | 100-200 | 100-200 | 6.5-7.5 |
| 11 | 180-220 | 300-400 | 6.5-7.5 |
| 12 | 180-220 | 200-300 | 6.5-7.5 |
| 13 | 180-220 | 100-200 | 6.5-7.5 |
| 14 | 180-220 | 100-200 | 6.9-7.1 |
| 15 | 180-220 | 100-200 | 7.0 |

As indicated above, in certain embodiments, the one or more First Channels are part of a network of interconnecting channels. In the context of such a network, one embodiment provides that at least 90% by volume of the channels in the network have a height in the range of about 50 µm to about 300 µm, and a width in the range of about 50 µm to about 900 µm.

### Shear Rate

The First Channel(s) can be characterized according to the fluid shear rate observed as a solution travels through the First Channel(s). In certain embodiments, the one or more First Channels are characterized as having a fluid shear rate in the range of about 100 s⁻¹ to about 4000 s⁻¹ for blood at 37.0 °C, a range of about 100 s⁻¹ to about 3000 s⁻¹ for blood at 37.0 °C, a range of about 400 S⁻¹ to about 2200 s⁻¹ for blood at 37.0 °C, a range of about 1000 s⁻¹ to about 2200 s⁻¹ for blood at 37.0 °C, a range of about 1500 s⁻¹ to about 2200 s⁻¹ for blood at 37.0 °C, or a range of about 1900 s⁻¹ to about 2200 s⁻¹ for blood at 37.0 °C.

### Quantify of Fluid Transport

The First Channel(s) can be further characterized according to quantity of fluid that can be transported through a population of said channels. For example, in certain embodiments, a population of 8000 to 9000 First Channels can transport blood at a rate of about 1 mL/min to about 500 mL/min, about 15 mL/min to about 150 mL/min, about 50 mL/min to about 100 mL/min, about 100 mL/min to about 150 mL/min, or about 15 mL/min to about 50 mL/min. In certain other embodiments, the microfluidic device contains a plurality of First Channels that, collectively, are configured to transport fluid in an amount of about 15 mL/min to about 150 mL/min through said plurality of First Channels.

### Amount of Fluid Transfer Through the Filtration Membrane

The First Channel(s) can be further characterized according to amount of fluid that passes from the First Channel(s) through the filtration membrane to the Second Channel(s). In certain embodiments, the one or more First Channels are configured so that the amount of fluid that passes through the filtration membrane covering the at least one First Channel is in the range of about 3% v/v to about 50% v/v of the fluid that enters the at least one First Channel. In certain embodiments, the one or more First Channels are configured so that the amount of fluid that passes through the filtration membrane covering the at least one First Channel is in the range of about 10% v/v to about 25% v/v of the fluid that enters the at least one First Channel.

### B. Features of the Second Channel

The Second Channel(s) is positioned on the opposite side of the filtration membrane from the First Channels) and is in fluid communication with at least one First Channel via the filtration membrane, i.e., the Second Channel(s) is complimentary to the one or more First Channels. The Second Channel(s) may have different height and width features compared to the First Channel(s). In certain embodiments, the Second Channel(s) is wide enough to cover ten or more First Channels, such as it covers 10, or 15 First Channels.

In certain embodiments, at least one Second Channel has a width in the range of about 50 µm to about 900 µm, and a length in the range of about 3 cm to about 20 cm. In certain other embodiments, the at least one Second Channel has a width in the range of about 50 µm to about 900 µm, and a length in the range of about 3 cm to about 10 cm. In certain other embodiments, the at least one Second Channel has a width in the range of about 50 µm to about 500 µm. In certain embodiments, at least one Second Channel has a length in the range of about 6 cm to about 8 cm, or a length of about 7 cm.

In other examples, the Second Channel(s) may be exact mirror image of the First Channel(s) and located precisely thereover, or may instead take another suitable form (e.g., a single Channel coextensive with and located opposite the network of First Channels across the membrane).

### II. Filtration Membrane

The filtration membrane can be selected to achieve separation of particular analytes. The filtration membrane preferably is porous and at least semi-permeable. A variety offiltration membranes are known in the art, and are contemplated to be amenable for use in the microfluidic devices described herein.

The membrane pore structure and size determine which of the fluids/solutes pass through to the Second Channel(s) and reservoir. In certain embodiments, the membrane thickness may range from approximately 1 µm to approximately 500 µm. In certain embodiments, the membrane thickness may range from approximately 100 µm, from approximately 100 µm to approximately 200 µm, from approximately 200 µm to approximately 300 µm, or from approximately 230 µm to approximately 270 µm.

To allow mass transfer across the membrane, the membrane, or at least a portion thereof, should be permeable or semi-permeable (i.e., selectively permeable to some, but not to other ions and molecules). Permeability may be achieved by using a semi-porous or porous material (such as polyethersulfone), whereby mass transfer takes place through the pores.

Pores in the membrane may be formed through processes such as track etching, solute leaching, solvent degradation, selective etching, molding, or phase inversion techniques. Membrane pore sizes may range from 0 - 100 nm and may be chosen to select retention of particular solutes and removal of other solutes. In addition to liquid or water to be removed from the blood, the membrane may also allow removal of ions including potassium, sodium, chlorine, and magnesium, small molecules such as urea and creatinine, and middle molecules such as β2-microglobulin. In general, the membrane will enable retention of proteins such as albumin, fibrin, and fibronectin, and cells in the blood.

Exemplary membrane materials include polyethersulfone, polycarbonate, polyimide, silicon, cellulose, PolyDiMethylSiloxane (PDMS), PolyMethylMethacrylate (PMMA), PolySulfone (PS), PolyCarbonate (PC), or from a degradable material such as PLGA, PolyCaproLactone (PCL) or Biorubber. In certain embodiments, the membrane comprises a polyethersulfone.

### III. Fluid Conduits and Pumps

The microfluidic devices described herein may optionally contain one or more of: (i) a first access conduit affording fluid communication with an input end of one or more First Channels; (ii) a first return conduit affording fluid communication with an output end of one or more First Channels; (iii) a second return conduit affording fluid communication with an output end of the at least one Second Channel; and (iv) a pump for ensuring that a fluid entering the first access conduit flows through one or more First Channels and out the first return conduit.

Access and return conduits convey fluid, such as patient blood, to and from the First Channel(s) and Second Channel(s). Access may be through an IV needle, cannulae, fistula, catheter, or an implanted access device. The access points may be existing points for previous treatments (e.g., hemodialysis) and may be arterio-venous or veno-venous in nature. The conduits can be standard medical tube materials including polymers such as silicone rubber, polyurethane, polyethylene, polyvinyl chloride, and latex rubber. An approximate size range of the inner diameter of the access conduits can be 300 µm - 1 cm. The access conduits can be integrated into the microfluidic device, or can instead be separate and have attachment points to connect to the microfluidic device.

A pump may regulate blood flow rate into the device, e.g., if arterial blood pressure is not high enough for the particular application or if a venous-venous access is deemed more desirable. In some cases, a physiological blood pressure of 120 mmHg may be sufficient to drive blood flow from an arterial access through the microfluidic device and back to the patient. In other cases, particularly where veno-venous access is used, a pump is used to drive blood through the microfluidic device. Pump flow and pressure, along with membrane porosity and channel geometry, determine the rate at which the fluids/solutes are extracted. Increased pump pressure increases the rate of convection of blood liquids and solutes across the membrane and into the reservoir. In addition, increased pump pressure drives a higher flow through the microfluidic device. Although optimal pump pressure depends on the desired blood flow and filtration rates, pump pressures ranging from 0 - 650 mmHg are representative. Blood flow rates through the microfluidic device may be somewhat lower than typical renal blood flow of 1.5 L/min, e.g., in the range of 0 - 500 mL/min.

The use of micropump and microvalve technology can be used to control the rate of convective transfer of water, small molecules and proteins, and the delivery and distribution of leaching or sorbent molecules. Microflow sensors and other elements with the ability to control flow in a range of channel dimensions and architectures may also be used. The use of micropumps, microvalve, and microflow sensors are contemplated to be capable of operating for long periods on a single, small battery charge.

### IV. Reservoir for Fluid Storage

The microfluidic device may optionally comprise a reservoir for collecting filtrate extracted from the fluid via the filtration membrane. In certain embodiments, the reservoir has a volume that determines an amount of filtrate extracted from the fluid via the filtration membrane. In certain other embodiments, the reservoir is an extension of the at least one Second Channel. In certain other embodiments, the reservoir is fluidly coupled to the second return conduit.

The reservoirs (e.g., for water and other waste products from blood dialysis) may be removable so that the weight of the device and burden on the patient is minimized. The reservoir volume determines the amount of fluids/solutes extracted, as diffusion and convection are severely limited once the reservoir is full. Essentially, when the fixed-displacement reservoir is full, it provides backpressure to prevent further filtration of liquids from the blood and limits convective flow across the membrane. In addition, the concentration of solutes in the reservoir increases over time, thereby slowing the rate of diffusion of solutes across the membrane. The principal weight in the waste stream is comprised of water, since 95% of urine content by weight is water. Since typical urine output for adults is 1.5 L per day, if the microfluidic device removes about 50% of the water normally excreted on a daily basis, three 250 mL (∼ 9 oz.) water packets can be easily and safely removed from the device during a 24-hr period. Unlike an implantable water filtration unit, there is no need for an invasive fluid connection between the device and the urinary system, thereby eliminating the risk of infection and other surgical complications. Reservoirs can be sized according to such factors as patient mass, required liquid removal, number of reservoir changes desired per day, liquid intake by the patient, and patient blood pressure.

### V. Sorbent System

The microfluidic device can include one or more additional components such as a sorbent system to selectively bind certain compounds or absorb fluid for storage in the reservoir. The sorbent material can reside within the reservoir and simply absorb water, thereby stabilizing the fluid extracted from the patient. In another application, the sorbent material can specifically bind urea, creatinine or other solutes, in order to reduce their concentration in the filtrate and thereby remove a larger quantity of them from the blood.

### VI. Delivery System

Another component that may be incorporated into the microfluidic device is a delivery system - such as a degradable ion-leaching system, micropump-based device, or a porous reservoir - to administer substances (such as ions, anti-coagulant compositions, and/or nutrients) directly to the patient's bloodstream. The delivery system can release, for example, ions, into the blood within the device in a time-release format to replenish ions lost during filtration or to otherwise balance ion concentration in the patient. Ions can include sodium, magnesium, chlorine, and potassium. A degradable system can include the substance to be delivered encased in a degradable matrix. As water contacts the matrix, it degrades, thereby releasing the substance in a time-dependent manner. A micropump system can use the pump pressure to release a prescribed quantity of a liquid solution containing the substance. A porous reservoir, for example, allows passage of ions through a porous media via diffusion driven by a high concentration of the ions in the reservoir.

### VII. Microfluidic Device Containing Cells

The device may be further enhanced by including cells on the inner structures of the device, specifically lining the walls of the channels. Accordingly, one aspect of the invention relates to microfluidic devices described herein where a cell is adhered to an inner wall of at least one of the channels. The cells may be human or other mammalian cells, and may, for example, be sourced from kidney tissue to include general parenchymal cells of the kidney, epithelial cells, endothelial cells, progenitor cells, stem cells, or epithelial cells from the nephron and its structures (such as the proximal tubule and loop of Henle). The cells may be primary isolates from tissue, patient biopsy cells, commercial cell lines, or engineered cells from various sources.

### VIII. Preparation of Microfluidic Device

Microfluidic devices described herein can be prepared by securing a filtration membrane between two polymeric devices having one or more channels on the surface of the polymeric device. The two polymeric devices are oriented so that the surface from each polymer device bearing the channels is attached to the filtration membrane and the channel(s) from the first polymer device are aligned to overlap with the channel(s) from the second polymer device, as illustrated in Figure 6. The filtration membrane (i.e., porous membrane identified in Figure 6) may be adhered to the polymer devices using plasma bonding, adhesive bonding, thermal bonding, cross-linking, mechanical clamping, or combinations of the foregoing.

The polymeric device may be prepared using techniques known in the art, such as by molding channel structures in a polymer using a mold created through a microfabrication technique. The mold can be patterned through photolithography or electron-beam lithography and etched using a wet etch, solvent etch, or dry etching procedure such as reactive ion etching. The mold may be fabricated in silicon, silicon dioxide, nitride, glass, quartz, metal, or a photoresist adhered to a substrate. The mold may optionally be converted to a more durable form through replication in polydimethylsiloxane, epoxy, or metal. The mold can then be used to mold the polymer structures of the device such as the large access conduits or the smaller channels for blood or filtrate flow. Molding may be accomplished through hot-embossing, injection molding, casting, or other conventional replication procedures.

In certain embodiments, microfluidic devices are manufactured from hard polymers (or "hard plastics"). Suitable hard polymer materials include thermoset polymers such as, for example, polyimide, polyurethane, epoxies, and hard rubbers, as well as thermoplastic polymers such as, for example, polystyrene, polydimethylsiloxane, polycarbonate, poly(methyl methacrylate), cyclic olefin copolymer, polyethylene, polyethylene terephthalate (PET), polyurethane, polycaproleacton (PCA), polyactic acid (PLA), polyglycolic acid (PGA), and poly(lactic-co-glycolic acid) (PGLA). Some of these materials (e.g., PCA, PLA, PGA, and PGLA) are biodegradable, and therefore also suitable for tissue engineering applications.

Cyclic olefin copolymer (COC) can be used, and it has good optical, chemical, and bulk properties. For example, COC exhibits strong chemical resistance and low water absorption, which are important characteristics for devices often sterilized in chemical solvents and used in aqueous environments.

Hard polymer materials facilitate hot embossing (or, in some embodiments, cold embossing) methods for device fabrication. The process begins with the design and fabrication of a photomask defining the microchannels, followed by photolithographic patterning of a (for example, standard 4-inch) silicon wafer coated with photoresist. In one embodiment, the patterning step involves spin-coating the pre-baked, clean silicon wafer with SU8 photoresist (available, e.g., from MicroChem, MA, USA) twice at 2000 rpm for 30 seconds; placing the photomask onto the wafer with a mask aligner (e.g., Karl Suss MA-6; Suss America, Waterbury, VT) and exposing the wafer to UV light; developing the wafer for 12 minutes in a developer (e.g., Shipley AZ400K); and baking the wafer at 150 °C for 15 minutes. In the resulting SU8 pattern, the microchannels correspond to raised features having, in one embodiment, a height of 110 µm ± 10 µm.

The patterned SU8 photoresist serves as a mold to create a second, negative replica cast mold of PDMS (e.g., Sylgard 184 from Dow Chemical, MI, USA) (step 306). In one embodiment, the PDMS base elastomer and curing agent are mixed in a 10:1 ratio by mass, poured on the patterned SU8 wafer, placed under vacuum for about 30 minutes to degas, and cured in an oven at 80 °C for more than 2 hours. In the PDMS mold, the channels are recessed. A durable epoxy master mold may subsequently be created from the PDMS mold. In one embodiment, this is accomplished by mixing Conapoxy (FR-1080, Cytec Industries Inc., Olean, NY, USA) in a 3:2 volume ratio of resin and curing agent, pouring the mixture into the PDMS mold, and curing it at 120 °C for 6 hours.

The cured epoxy master is then released from the PDMS mold, and hot-embossed into a COC or other thermoplastic substrate to form the microfluidic features. The embossing step is typically carried out under load and elevated temperatures, for example in a press that facilitates controlling the temperature via a thermocoupler and heater control system, and applying pressure via compressed air and vacuum. Temperature, pressure, and the duration of their application while the epoxy master mold is in direct contact with the substrate constitute manufacturing parameters that may be selected to optimize the fidelity of the embossed features, and the ability to release and mechanical properties of the embossed layers. In one embodiment, the COC (or other thermoplastic) plate is placed on the epoxy master, loaded into the press, and embossed at 100 kPa and 120 °C for one hour. The resulting embossed plates are then cooled to 60°C under 100 kPa pressure, unloaded from the press, and separated from the epoxy master mold.

A durable master mold that can withstand high temperatures and pressures and serves as a stamp for embossing the microfluidic pattern into the thermoplastic wafer need not necessarily be made from epoxy. In alternative embodiments, etched silicon or electroformed or micromachined metal (e.g., nickel) molds may be used. Epoxy masters are advantageous because they are not only durable, but also comparatively inexpensive to fabricate.

When the microfluidic device contains a pump, the pump may be integral to the device, as in a flexible section of channel used as a peristaltic pump, or a separate component assembled into the microfluidic device. The microfluidic device itself may be flexible, and once the molded polymers are bonded to the membrane the microfluidic device can be folded, rolled, or otherwise shaped to provide a compact and convenient form factor. Access conduits can be attached to channels in the microfluidic device or integrated into the microfluidic device during the molding process.

### IX. Application of Microfluidic Filtration Device in a Kidney Augmentation Device

The microfluidic devices described herein may be incorporated into a kidney augmentation device (KAD). In certain embodiments, the KAD provides enough augmentation of native kidney function for a patient to avoid, reduce or supplement dialysis for short term periods. Unlike conventional dialysis, the KAD allows full patient mobility while providing a gentler and more physiologic rate of dialysis over a longer time period. It may not be necessary to provide as much kidney function as a traditional dialysis system, since in various embodiments, the KAD is intended for relatively short periods of operation to either replace or supplement some dialysis sessions. Compared to a wearable dialysis system, the KAD is simpler and more compact, thereby reducing patient risk, cost and complexity, and allowing greater patient mobility.

In certain embodiments, the KAD may take the form of a microfluidic device comprising one or more First Channels and one or more Second Channels complementary to the First Channel(s); a filtration membrane separating the first and Second Channel(s); a first access conduit affording fluid communication with an input end of the First Channel(s); a first return conduit affording fluid communication with an output end of the First Channel(s); a second return conduit affording fluid communication with an output end of the Second Channel(s); a pump for ensuring that a fluid entering the first access conduit flows through the First Channel(s) and out the first return conduit; and a reservoir for collecting filtrate extracted from the fluid via the filtration membrane.

The device may have a second access conduit affording fluid communication with an input end of the Second Channel(s). In some embodiments, there are a plurality of First Channels taking the form of a network. For example, these may comprise branching channels having diameters or channel widths no greater than 500 µm. The Second Channels may be exact mirror images of the First Channels and located precisely thereover, or may instead take another suitable form (e.g., a single channel coextensive with and located opposite the network of First Channels across the membrane). In other embodiments, the First Channel(s) are a single wide but shallow channel having a ratio of width to height of at least 100, e.g., 7 - 10 µm deep and several mm wide.

The network of microchannels conveys blood and collects filtrate. The microchannels extend from the blood-access conduits and may take the form of a branching network, bifurcations, or other geometries to direct flow from the large-diameter blood-access conduits down to the microchannels, which can have channel widths or diameters ranging from 7 - 500 µm. The small channel widths decrease the diffusion distance for the fluids and solutes in the blood in order to improve transport from within the channel to the filtration membrane.

The channels may formed in a flexible matrix such that the device exhibits flexibility. The membrane is typically porous and at least semi-permeable. The membrane may have variable properties over its area, and there may be more than one membrane (each, for example, having a different selectivity).

The reservoir is desirably configured such that its volume determines an amount of fluids and solutes extracted from the fluid via the filtration membrane. The reservoir may be an extension of the Second Channel(s), or may be a separate structure fluidly coupled to the second return conduit.

A representative embodiment of the KAD comprises access and return conduits for a patient's blood supply, a pump to propel fluid through the device as needed, a network of microchannels for blood flow, a second set of channels to collect the extracted fluids/solutes, a membrane separating the first and second set of channels, and a reservoir to collect the extracted fluids/solutes. The patient's blood flows through the conduit and into the small channels. There, the fluids/solutes are extracted from the blood across the membrane via diffusion and convection into the second set of channels. The extracted fluids/solutes then flow to a reservoir for temporary storage. The filtered blood continues to flow through the small channels which connect to the return line and return the blood to the patient.

In certain embodiments, the KAD comprises a microfluidic device that comprises (i) at least one First Channel and at least one Second Channels complementary to the at least one First Channel; (ii) a filtration membrane separating the at least one First Channel from the at least one Second Channel; (iii) a first access conduit affording fluid communication with an input end of the at least one First Channel; (iv) a first return conduit affording fluid communication with an output end of the at least one First Channel; (v) a second return conduit affording fluid communication with an output end of the at least one Second Channel; (vi) a pump for ensuring that a fluid entering the first access conduit flows through the at least one First Channel and out the first return conduit; and (vii) a reservoir for collecting filtrate extracted from the fluid via the filtration membrane. In certain embodiments, at least the at least one First Channel comprises a plurality of channels in the form of a network. In certain embodiments, at least First Channels comprise branching channels having diameters or channel widths no greater than 500 µm. In certain embodiments, the membrane is porous and at least semi-permeable. In certain embodiments, the reservoir has a volume that determines an amount of filtrate extracted from the fluid via the filtration membrane. In certain embodiments, the device further comprises a sorbent system. In certain embodiments, the device further comprises a delivery system. In certain embodiments, the device further comprises cells adhered to inner walls of at least one of the channels. In certain embodiments, the channels are formed in a flexible matrix such that the device exhibits flexibility. In certain embodiments, the device further comprises a second access conduit affording fluid communication with an input end of the at least one Second Channel. In certain embodiments, the reservoir is an extension of the at least one Second Channel. In certain embodiments, the reservoir is fluidly coupled to the second return conduit. In certain embodiments, the at least one First Channel is a single wide but shallow channel having a ratio of width to height of at least 100.

The KAD may be compact and, in some embodiments, can be worn on the patients arm, leg, or torso and can be strapped to the patient to prevent movement and the possibility of removal of the blood access points. Blood access can be through arm or leg arteries and veins, as well as larger blood vessels in the torso. Installation of the KAD can be done at a dialysis clinic or doctors office, as well as by the patient themselves.

Advantages of the KAD include patient mobility; simple design leading to low cost; slower, gentler rate of dialysis relative to conventional approaches, which increases efficacy and reduces side effects; may allow reduction in dialysis sessions for a healthcare cost savings; decreased risk to patient due to simple design; improved patient outcomes due to more frequent and gentler treatment relative to conventional alternatives; better control of fluid flow and reduction in deleterious blood-device interactions due to the microfabricated channels; management of ions through time-release delivery system; and disposable/removable reservoir for filtrate allows a low-cost and simple means of disposal and regulation of volume of filtrate removed from the patient's blood.

In certain embodiments, the invention provides a wearable kidney augmentation device, comprising: (i) a filtration component comprising: (a) at least one First Channel and at least one Second Channel complementary to the at least one First Channel; and (b) a filtration membrane separating the at least one First Channel from the at least one Second Channel; wherein the at least one First Channel is configured to provide a fluid shear rate in the range of about 100 s⁻¹ to about 3000 s⁻¹ for blood at 37.0 °C; (ii) a first access conduit affording fluid communication with an input end of the at least one First Channel; (iii) a first return conduit affording fluid communication with an output end of the at least one First Channel; and (iv) a second return conduit affording fluid communication with an output end of the at least one Second Channel.

In certain embodiments, the one or more First Channels are characterized as having a fluid shear rate in the range of about 400 s⁻¹ to about 2200 s⁻¹ for blood at 37.0 °C, a range of about 1000 s⁻¹ to about 2200 s⁻¹ for blood at 37.0 °C, a range of about 1500 s⁻¹ to about 2200 s⁻¹ for blood at 37.0 °C, or a range of about 1900 s⁻¹ to about 2200 s⁻¹ for blood at 37.0 °C. In certain embodiments, the wearable kidney augmentation device has at least one First Channel that is configured so that the amount of fluid that passes through the filtration membrane covering the at least one First Channel is in the range of about 3% v/v to about 50% v/v of the fluid that enters the at least one First Channel. In certain embodiments, the wearable kidney augmentation device has at least one First Channel that is configured so that the amount of fluid that passes through the filtration membrane covering the at least one First Channel is in the range of about 10% v/v to about 25% v/v of the fluid that enters the at least one First Channel. In certain embodiments, the wearable kidney augmentation device contains a plurality of First Channels that, collectively, are configured to transport fluid in an amount of about 1 mL/min to about 500 mL/min through said plurality of First Channels. In certain embodiments, the wearable kidney augmentation device further comprises a pump for ensuring that a fluid entering the first access conduit flows through the at least one First Channel and out the first return conduit. In certain embodiments, the wearable kidney augmentation device further comprises a reservoir for collecting filtrate extracted from the fluid via the filtration membrane.

### X. Use of Microfluidic Device for Delivering Fluids to Blood

Microfluidic devices described herein may also be used for delivering a fluid to blood or other liquids. For example, while blood is passing through a First Channel, a fluid could be applied to a Second Channel under conditions such that the fluid passes through the filtration membrane to enter the First Channel and mix with blood passing through the First Channel. The fluid could be applied to the Second Channel under pressure so that the fluid passes from the Second Channel through the filtration membrane to enter the First Channel. In such circumstances, a pump may be connected to the Second Channel to deliver the fluid under pressure. Alternatively, the fluid may pass from the Second Channel through the filtration membrane to enter the First Channel due to an analyte concentration gradient or other means.

### XI. Methods of Filtering a Liquid Solution

Another aspect of the invention provides a method of filtering a liquid solution containing an analyte to provide a purified solution containing less analytes than said liquid solution. The method comprises the steps of: (i) introducing said liquid solution containing said analyte into the input end of one or more First Channels of the device described herein and configured with a filtration membrane that is at least semi-permeable to said analyte; and (ii) collecting the purified liquid solution from the output end of one or more First Channels.

In certain embodiments, the liquid solution is blood. In certain embodiments, the analyte is urea, uric acid, creatinine, or a mixture thereof. In certain embodiments, the analyte is water, an alkali metal ion, or an alkaline earth metal ion.

### EQUIVALENTS

The invention may be embodied in other specific forms. The foregoing embodiments are therefore to be considered in all respects illustrative rather than limiting the invention described herein. Scope of the invention is thus indicated by the appended claims rather than by the foregoing description, and all changes that come within the meaning and range of equivalency of the claims are intended to be embraced therein.

## Claims

1. A microfluidic device, comprising:
(i) ten or more First Channels (100), each of the ten or more First Channels (100) having a height in the range of about 50 µm to about 500 µm, a width in the range of about 50 µm to about 900 µm, a height to width ratio in the range of 1:1 to about 1:4, and a length in the range of about 3 cm to about 20 cm;
(ii) at least one Second Channel (101) complementary to the ten or more First Channels (100), wherein each of the at least one Second Channel has a width and the width of each of the at least one Second Channel (101) spans at least ten of the ten or more First Channels (100); and
(iii) a permeable filtration membrane (102), wherein the ten or more First Channels (100) are in fluid communication with the at least one Second Channel (101) through the permeable filtration membrane (102) separating the ten or more First Channels (100) from the at least one Second Channel (101).

2. The device of claim 1, wherein the ten or more First Channels (100) have a height to length ratio in the range of 1:250 to about 1:800.

3. The device of claim 1, wherein at least 90% by volume of the channels in the network have a height in the range of about 50 µm to about 300 µm, and a width in the range of about 50 µm to about 900 µm.

4. The device of claim 1, wherein the membrane (102) comprises a polyethersulfone.

5. The device of claim 1, further comprising:
(i) a first access conduit affording fluid communication with an input end of the ten or more First Channels (100);
(ii) a first return conduit affording fluid communication with an output end of the ten or more First Channels (100);
(iii) a second return conduit affording fluid communication with an output end of the at least one Second Channel (101); and
(iv) a pump for ensuring that a fluid entering the first access conduit flows through the ten or more First Channels (100) and out the first return conduit.

6. The device of claim 5, further comprising a reservoir for collecting filtrate extracted from the fluid via the filtration membrane (102), wherein the reservoir is an extension of the at least one Second Channel (101) and has a volume that determines an amount of filtrate extracted from the fluid via the filtration membrane (102).

7. The device of claim 1, further comprising a sorbent system.

8. The device of claim 1, further comprising at least one cell adhered to at least one inner wall of at least one of the channels.

9. The device of claim 1, wherein the ten or more First Channels (100) are configured to transport fluid in an amount of about 1 mL/min to about 500 mL/min through said ten or more First Channels (100).

10. A method of filtering a liquid solution containing an analyte to provide a purified solution containing less analyte than said liquid solution, the method comprising the steps of:
(i) providing a microfluidic device of any of the claims 1-9;
(ii) introducing said liquid solution containing said analyte into the input end of the ten or more First Channels (100) of the microfluidic device, wherein the filtration membrane (102) is at least semi-permeable to said analyte;
(iii) collecting the purified liquid solution from the output end of the ten or more of First Channels (100) of the microfluidic device,
wherein the method is not for treatment of a human or animal body.

11. The method of claim 10, wherein the liquid solution is blood and the analyte is urea, uric acid, creatinine, or a mixture thereof.

## Patentansprüche

1. Mikrofluidische Vorrichtung, Folgendes beinhaltend:
(i) zehn oder mehr Erste Kanäle (100), wobei jeder der zehn oder mehr Ersten Kanäle (100) eine Höhe im Bereich von etwa 50 µm bis etwa 500 µm, eine Weite im Bereich von etwa 50 µm bis etwa 900 µm, ein Höhe-zu-Weite-Verhältnis im Bereich von 1 zu 1 bis etwa 1 zu 4, und eine Länge im Bereich von etwa 3 cm bis etwa 20 cm besitzt;
(ii) mindestens einen Zweiten Kanal (101), ergänzend zu den zehn oder mehr Ersten Kanälen (100), wobei jeder von dem mindestens einen Zweiten Kanal eine Weite hat und die Weite eines jeden des mindestens einen Zweiten Kanals (101) mindestens zehn der zehn oder mehr Ersten Kanäle (100) misst; und
(iii) eine durchlässige Filtermembran (102), wobei die zehn oder mehr Ersten Kanäle (100) in Fluidkommunikation mit dem mindestens einen Zweiten Kanal (101) durch die durchlässige Filtermembran (102) stehen, welche die zehn oder mehr Ersten Kanäle (100) von dem mindestens einen Zweiten Kanal (101) trennt.

2. Vorrichtung nach Anspruch 1, bei welcher die zehn oder mehr Ersten Kanäle (100) ein Höhe-zu-Länge-Verhältnis im Bereich von 1 zu 250 bis etwa 1 zu 800 besitzen.

3. Vorrichtung nach Anspruch 1, bei welcher mindestens 90 Volumenprozent der Kanäle im Netzwerk eine Höhe im Bereich von etwa 50 µm bis etwa 300 µm, und eine Weite im Bereich von etwa 50 µm bis etwa 900 µm besitzen.

4. Vorrichtung nach Anspruch 1, bei welcher die Membran (102) ein Polyethersulfon beinhaltet.

5. Vorrichtung nach Anspruch 1, weiterhin beinhaltend:
(i) eine erste Zugangsleitung, welche Fluidkommunikation mit einem Eingangsende der zehn oder mehr Ersten Kanäle (100) herstellt;
(ii) eine erste Rücklaufleitung, welche Fluidkommunikation mit einem Ausgangsende der zehn oder mehr Ersten Kanäle (100) herstellt;
(iii) eine zweite Rücklaufleitung, welche Fluidkommunikation mit einem Ausgangsende des mindestens einen Zweiten Kanals (101) herstellt; und
(iv) eine Pumpe zur Gewährleistung, dass ein in die erste Zugangsleitung eintretendes Fluid durch die zehn oder mehr Ersten Kanäle (100) hindurch und aus der ersten Rücklaufleitung heraus fließt.

6. Vorrichtung nach Anspruch 5, weiterhin beinhaltend einen Tank zur Sammlung von Filtrat, das aus dem Fluid über die Filtermembran (102) extrahiert wird, wobei der Tank eine Erweiterung von dem mindestens einen Zweiten Kanal (101) ist und ein Volumen besitzt, das eine Menge von aus dem Fluid über die Filtermembran (102) extrahierten Filtrats bestimmt.

7. Vorrichtung nach Anspruch 1, weiterhin ein Soiptionssystem beinhaltend.

8. Vorrichtung nach Anspruch 1, weiterhin mindestens eine Zelle beinhaltend, die an mindestens einer Innenwand von mindestens einem der Kanäle haftet.

9. Vorrichtung nach Anspruch 1, bei welcher die zehn oder mehr Ersten Kanäle (100) konfiguriert sind, um Fluid in einer Menge von etwa 1 mL/Min. bis etwa 500 mL/Min. durch die zehn oder mehr Ersten Kanäle (100) zu transportieren.

10. Verfahren zur Filterung einer ein Analyt enthaltenden flüssigen Lösung zur Lieferung einer gereinigten Lösung, welche weniger Analyt enthält als die flüssige Lösung, wobei das Verfahren folgende Schritte beinhaltet:
(i) Bereitstellen einer mikrofluidischen Vorrichtung nach einem der Ansprüche 1 bis 9;
(ii) Einbringen der das Analyt enthaltenden flüssigen Lösung in das Eingangsende der zehn oder mehr Ersten Kanäle (100) der mikrofluidischen Vorrichtung, wobei die Filtermembran (102) mindestens halbdurchlässig gegenüber dem Analyt ist;
(iii) Sammeln der gereinigten flüssigen Lösung vom Ausgangsende der zehn oder mehr Ersten Kanäle (100) der mikrofluidischen Vorrichtung,
wobei das Verfahren nicht zur Behandlung eines menschlichen oder tierischen Körpers bestimmt ist.

11. Verfahren nach Anspruch 10, bei welchem die flüssige Lösung Blut und der Analyt Harnstoff, Harnsäure, Kreatinin oder eine Mischung daraus ist.

## Revendications

1. Dispositif microfluidique, comprenant:
(i) au moins dix Premiers Canaux (100), chacun de ces dix ou plus Premiers Canaux (100) ayant une hauteur comprise entre environ 50 µm et environ 500 µm, une largeur comprise entre environ 50 µm et environ 900 µm, un rapport hauteur/largeur compris entre 1:1 et environ 1:4, et une longueur comprise entre environ 3 cm et environ 20 cm ;
(ii) au moins un Second Canal (101) complémentaire des dix ou plus Premiers Canaux (100), chacun de l'au moins un Second Canal (101) ayant une largeur, la largeur de chacun de l'au moins un Second Canal (101) étant au moins égale à la largeur de dix des dix ou plus Premiers Canaux (100) ; et
(iii) une membrane de filtration perméable (102), où les dix ou plus Premiers Canaux (100) sont en communication fluidique avec ledit au moins un Second Canal (101), à travers la membrane de filtration perméable (102) séparant les dix ou plus Premiers Canaux (100) de l'au moins un Second Canal (101).

2. Dispositif selon la revendication 1, dans lequel les dix ou plus Premiers Canaux (100) ont un rapport hauteur/longueur compris entre 1:250 et environ 1:800.

3. Dispositif selon la revendication 1, dans lequel au moins 90 % en volume des canaux contenus dans le réseau ont une hauteur comprise entre environ 50 µm et environ 300 µm, et une largeur comprise entre environ 50 µm et environ 900 µm.

4. Dispositif selon la revendication 1, dans lequel la membrane (102) comprend une polyéthersulfone.

5. Dispositif selon la revendication 1, comprenant par ailleurs :
(i) un premier conduit d'accès permettant une communication fluidique avec une extrémité d'entrée des dix ou plus Premiers Canaux (100) ;
(ii) un premier conduit de retour permettant une communication fluidique avec une extrémité de sortie des dix ou plus Premiers Canaux (100) ;
(iii) un deuxième conduit de retour permettant une communication fluidique avec une extrémité de sortie de l'au moins un Second Canal (101) ; et
(iv) une pompe pour veiller à ce qu'un fluide entrant dans le premier conduit d'accès s'écoule à travers les dix ou plus Premiers Canaux (100) et en sorte par le premier conduit de retour.

6. Dispositif selon la revendication 5, comprenant par ailleurs un réservoir servant à recueillir le filtrat extrait du fluide via la membrane de filtration (102), ce réservoir étant dans le prolongement de l'au moins un Second Canal (101) et ayant un volume déterminant une quantité de filtrat extrait du fluide via la membrane de filtration (102).

7. Dispositif selon la revendication 1, comprenant par ailleurs un système adsorbant.

8. Dispositif selon la revendication 1, comprenant par ailleurs au moins une cellule adhérant à au moins une paroi interne d'au moins un des canaux.

9. Dispositif selon la revendication 1, dans lequel les dix ou plus Premiers Canaux (100) sont configurés de manière à transporter une quantité de fluide d'environ 1 mL/min à environ 500 mL/min à travers lesdits dix ou plus Premiers Canaux (100).

10. Méthode de filtration d'une solution liquide contenant un analyte, visant à produire une solution purifiée contenant moins d'analyte que ladite solution liquide, cette méthode comprenant les étapes suivantes :
(i) fournir un dispositif microfluidique selon l'une quelconque des revendications 1 à 9 ;
(ii) introduire ladite solution liquide contenant ledit analyte dans l'extrémité d'entrée des dix ou plus Premiers Canaux (100) du dispositif microfluidique, dans lequel la membrane de filtration (102) est au moins semi-perméable audit analyte ;
(iii) recueillir la solution liquide purifiée à partir de l'extrémité de sortie des dix ou plus Premiers Canaux (100) du dispositif microfluidique,
la méthode n'étant pas destinée au traitement d'un corps humain ou animal.

11. Méthode selon la revendication 10, dans laquelle la solution liquide est le sang et l'analyte est l'urée, l'acide urique, la créatinine, ou un mélange de ces composés.
